# EUROPEAN PATENT SPECIFICATION

(11) **EP 3 513 163 B1**
(45) Date of publication and mention of the grant of the patent: **07.08.2024**
(21) Application number: 18799868.7
(22) Date of filing: 31.10.2018
(51) Int. Cl.: G01N 1/38

(54) **SYSTEMS AND METHODS FOR BIOLOGICAL SAMPLE LABORATORY SCREENING**
SYSTEME UND VERFAHREN ZUM LABORSCREENING BIOLOGISCHEN PROBEN
SYSTÈMES ET PROCÉDÉS DE CRIBLAGE EN LABORATOIRE D'ÉCHANTILLONS BIOLOGIQUES

(30) Priority: 22.11.2017 US 201762589957 P
(43) Date of publication of application: 24.07.2019
(73) Proprietor: Grifols Diagnostic Solutions Inc., Emeryville, CA 94608 (US)
(72) Inventor: TRAN, Quang, Fremont, California 94555 (US); WUMMER, Joel, Castro Valley, California 94552 (US); CODY, Dennis, El Cerrito, California 94530 (US)
(74) Representative: Durán-Corretjer, S.L.P.
(86) International application number: PCT/IB2018/058536
(87) International publication number: WO 2019/102282

(56) References cited:
- EP-A1- 2 393 935
- EP-A1- 3 109 641
- EP-A1- 3 343 822
- EP-A2- 2 211 165
- WO-A1-2017/038250
- US-A1- 2013 316 461

## Description

### BACKGROUND

### Field

The present disclosure relates to apparatuses, systems, and methods for biological sample laboratory screening, including, for example, monitoring operations, tracking progress, and generating progress indications.

### Description of the Related Art

Biological (blood and plasma) screening laboratories and clinical diagnostic laboratory testing has been and continues to be a very complex process with multiple devices that contain multiple assay types. Each assay type and device can require a unique workflow, causing increased complexity in screening and diagnostic laboratory procedures.

There can be a large number of steps and "islands" of sub-workflow steps that laboratory technicians may need to manage. Regulatory agencies require screening laboratories to analyze all biological test results from multiple devices and then determine a conclusion for that biological sample. The process of collecting all the biological test results for evaluation and determining a conclusion for a biological sample can include many steps, several which are traditionally performed manually by a technician. Furthermore, laboratory technicians traditionally track elements, states, and workflows by physically checking each step and "island" of sub-workflow steps. Since laboratory technicians manually keep records up-to-date, tracking the status of all devices and samples become burdensome and information can quickly become outdated. Laboratory technicians also do not have an effective way of searching for the current state of a sample, device, and the like. As a result of the quickly outdated records and the lack of effective searching of current states, current blood and/or plasma screening labs have a difficult time preventing duplicate testing of samples.

Validation and accuracy of results can be of utmost importance in this type of environment. In addition, many business factors are involved when considering management of the biological sample screening labor, costs associated with processing the samples, and speed and efficiency of getting results so that product may be used.

EP 2393935 A1 refers to a method for the detection or diagnosis of a bacterial infection or colonization, in particular, to the detection of the presence or absence of particular bacteria using mass spectrometric analysis, in particular MALDI-TOF mass spectrometric analysis, wherein the enrichment process of the said method comprises a centrifugation and pooling protocol. However, the above-mentioned problems still remain unsolved.

### SUMMARY

The invention provides for a system and a method according to the appended set of claims.

### BRIEF DESCRIPTION OF THE DRAWINGS

Embodiments of the present disclosure will now be described hereinafter, by way of example only, with reference to the accompanying drawings in which:
FIG. 1 illustrates a biological sample screening workflow according to some embodiments;
FIG. 2 illustrates a screening workflow system according to some embodiments;
FIG. 3 illustrates a graphical user interface displaying workflows according to some embodiments;
FIG. 4 illustrates data used for managing and scheduling workflow according to some embodiments;
FIG. 5 illustrates a process for managing test orders;
FIG. 6 illustrates a process for managing the release of test results according to some embodiments;
FIG. 7 illustrates a process for evaluating potential constituents of a pool to prevent unwanted pools or tests according to some embodiments;
FIG. 8 illustrates a process for determining if constituents in a pool are valid before testing according to some embodiments;
FIG. 9 illustrates a process for deconvoluting a reactive pool according to some embodiments.
FIG. 10 illustrates a display of a list of actions for an operator to ensure efficient operation of the system and release of results according to some embodiments.

### DETAILED DESCRIPTION

Embodiments disclosed herein relate to apparatuses and methods of managing biological sample, such as blood and/or plasma sample, screening laboratory workflow, for example monitoring operations, tracking progress, and generating progress indications. A biological sample, such as blood and/or plasma sample, screening laboratory workflow process can include manual procedures to support the process of screening and performing diagnostics in the laboratory. There is a need to create, automate, and/or track these and other procedures for faster and accurate processing and managing.

A blood and/or plasma screening laboratory workflow system can determine and/or track the current state of devices and display the states on a graphical user interface illustrating a plurality of workflows. The graphical user interface can enable real time information to technicians of current operating states for each biological sample, each assay, and/or each device. Such a graphical user interface can enable a technician to directly observe a particular device and/or a biological sample without the need of manually identifying the stage in the workflow for the biological sample.

The blood and/or plasma screening laboratory workflow system can provide a chronology of states and results for biological samples. For example, the blood and/or plasma screening laboratory workflow system can display device states, biological sample test results, and workflows associated with a particular biological sample. The blood and/or plasma screening laboratory workflow system can also display the current state of the biological sample. The system can provide a chronology of states, display the status of instruments and biological samples, indicate what components need operator attention, and search and display the status of samples and tests. The system can prevent unintended use, including duplicate testing.

The blood and/or plasma screening laboratory workflow system can implement a graphical user interface display displaying the status of the systems according to corresponding workflows. The status of the systems and the workflows of the blood and/or plasma screening and diagnostic laboratory can be stored and retrieved in a database. The blood and/or plasma screening laboratory workflow system can evaluate data received from the various systems (for example, the accession system, the centrifuge system, etc.) and determine their current state to display on the graphical user interface.

The graphical user interface can indicate what operator actions are needed next to ensure efficient workflow. Actions can include replenishing assay kits, processing fluids, consumables or emptying waste containers among others. The list of actions required can be sorted by most urgent first, indicated by time or other priority metrics, and for one or more specific analyzers. Multiple analyzers that require the same type of attention and with the same urgency can be grouped and identified in the same graphical indicator.

The graphical user interface can include a search that can be used to list characteristics of a biological sample. Upon entering a biological sample, the blood and/or plasma screening laboratory workflow system can retrieve data related to the biological sample from the database. The graphical user interface can display workflows associated with the biological sample and/or results of analysis or procedures performed on the biological sample. The graphical user interface can display the current state of the biological sample in each of the workflow stages.

The graphical user interface can display current operating states of systems and/or the current state of a biological sample according to a chronological list of stages in a workflow. A technician can use the graphical user interface to display these statuses, and can select any of the workflow stages to get further details on the operating state of that particular stage and/or corresponding system.

Some embodiments of the present disclosure provide a graphical user interface that allows technicians to effectively and accurately track biological samples and workflows at lower costs while meeting statutory regulations, such as for example regulations defined by the FDA, ISO, or CLIA.

### Biological Screening Workflow Overview

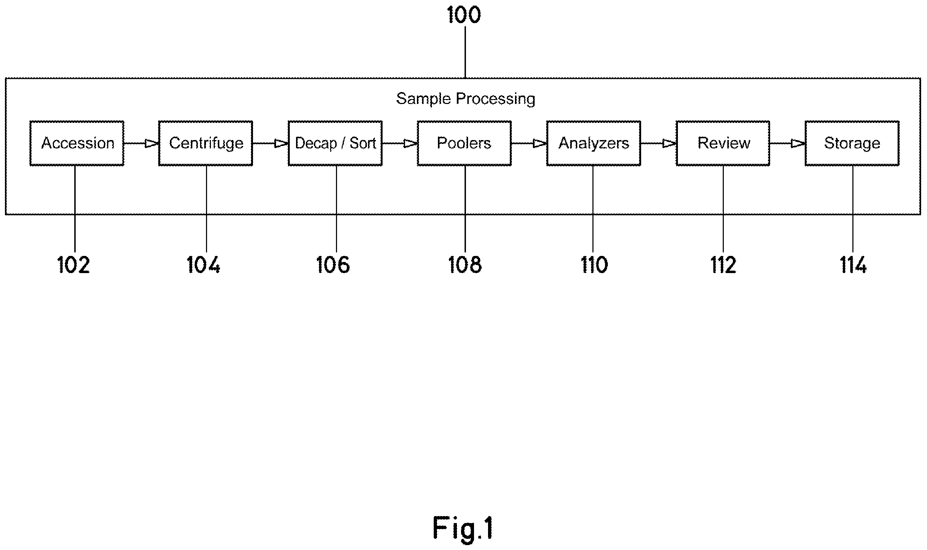

FIG. 1 illustrates a workflow 100 for biological sample screening, such as blood and/or plasma screening, according to some embodiments. The workflow 100 includes an accession stage 102, a centrifuge stage 104, a decap/sort stage 106, a pooler stage 108, an analyzer stage 110, a review stage 112, and a storage stage 114. The workflow 100 can be used, for example, for blood and/or plasma screening.

The accession system of the accession stage 102 can perform the following functions. An order of a test or of a group of tests can be received by the laboratory for a particular biological sample. When the laboratory receives the order and/or the biological sample, an accession number (such as a donation identification that can be scanned) and/or own specimen identifier can be assigned to the order, the test(s), and/or the biological sample. An identifier, such as a donation identifier, can include a number assigned via barcode kits to the donations at the collection center where the blood or plasma donation is made. Alternatively, the accession number and/or own specimen identifier can be a unique identifier generated by a computer that is assigned to the specimen. The accession number and/or the donation identifier can be used to identify the specimen as the specimen moves around the laboratory. For example, when the specimen is formally received by a laboratory or a health care service, an accession number and/or own specimen identifier can be placed on the specimen. The accession and/or own specimen identifier number can be based on information received from the order, used to identify tests for the specimen, information related to the biological sample, date and time of the collection of the specimen, information for billing purposes, and the like. Each collected specimen and/or group of specimens can be assigned its own accession number and/or own specimen identifier. A single accession number and/or own specimen identifier can be assigned for a group of biological samples (for example, samples of blood and/or plasma from the same patient have the same accession number), and/or an accession number and/or own specimen identifier can be assigned to each biological sample (for example, each sample of blood and/or plasma from the same patient has different accession numbers). In some embodiments, the biological sample is already associated with an identifier. In the accession stage 102, the accession system can access the identifier. For example, the specimen identifier can already be placed on the tube at the donation center. The accession system can read a barcode, a QR code, a unique identifier, a label, a code, an alphanumerical identifier, and the like. The laboratory can identify arrival of the specimen upon reading the identifier.

Accession numbers and/or own specimen identifiers can be used at various devices to identify when a biological sample has begun or completed the test at the device. The accession numbers and/or own specimen identifier can be used to keep track of each biological sample as it progresses through the workflow 100. The accession numbers and/or own specimen identifiers can also be used to assign certain biological samples to particular devices for a specific test during a time period. Thus, the device can read the accession number and/or own specimen identifier on the biological sample to identify whether the correct biological sample has arrived for testing. The device can send an updated state in the workflow 100 for the biological sample to the blood and/or plasma screening laboratory workflow system. The blood and/or plasma screening laboratory workflow system can then update the graphical user interface to show the latest status of the specimen in real time for a technician to assess.

The centrifuge system of the centrifuge stage 104 can perform the following functions. A centrifuge system can include equipment that places an object in rotation around a fixed axis and spins the object in a circle. The object can be a biological sample. The rotation applies a force perpendicular to the axis of the spin. The centrifuge system can separate some biological samples from a plurality of biological samples into a plurality of components.

At the centrifuge state 104, centrifuges can be used to separate components of the biological sample. The separation can be based on particle size or density. For example, the rotation causes the denser substances and particles to move outward in the radial direction. At the same time, the objects that are less dense are displaced and moved toward the center of the rotation. In a laboratory centrifuge, the denser particles can settle at the bottom of sample tubes while low-density substances rise to the top of the sample tubes.

In some implementations, a centrifuge separates blood into cells and either serum or plasma. The centrifuges can revolve at a specified number of revolutions per minute (RPM) for a certain time period. For example, a centrifuge can be assigned a particular biological sample to run at 1000 g RPM for 10 minutes, where g is the relative centrifugal force measured in multiples of the earth's gravitational field.

The biological sample can be assigned to a centrifuge at a particular time period. The centrifuge system can access the accession number and/or own specimen identifier of the biological sample and determine when the biological sample enters and leaves the centrifuge system. The centrifuge system can send the blood and/or plasma screening laboratory workflow system updated status of the biological sample to display real-time data to a technician. The blood and/or plasma screening laboratory workflow system can also determine whether the centrifuge system is available to receive another biological sample. Accordingly, the blood and/or plasma screening laboratory workflow system can adjust and optimize the workflows such that the appropriate biological sample is listed for the centrifuge system. For example, if one system took longer than expected to perform its test, the blood and/or plasma screening laboratory workflow system can automatically and dynamically adjust the workflows such that a different biological sample that is of higher priority is scheduled for the centrifuge system, earlier than the biological sample that was originally scheduled to be at the centrifuge system. The blood and/or plasma screening laboratory workflow system can adjust and/or optimize workflows based on the tracked status of one or more devices and/or samples. The blood and/or plasma screening laboratory workflow system can identify priority statuses for one or more devices and/or samples to factor into determining the adjustment and/or optimization. The blood and/or plasma screening laboratory workflow system can adjust and/or optimize workflows periodically and/or in response to an activity, such as the completion or delay of a test.

The decap/sorter system of the decap/sort stage 106 can perform the following functions. A decapper system can decap sample tubes containing biological samples for testing. For example, a decapper can automatically decap and/or cap screw cap tubes with internal or external threads. The sample tubes can then be separated from the screw caps, and be opened at the top for sampling for further testing. The decapper system can also turn the sample tubes before the decapping to improve decapping performance, and/or after decapping to prepare for the next phase in the workspace.

A sorter system can sort sample tubes containing the biological samples to determine the order and placement of the biological samples according to the optimized workflow. The sorter system can sort the sample tubes before and/or after the decapper system. For example, the sorter system can sort the tubes before the decapper system to reduce risk of cross contamination (e.g. if the decapper moves over opened tubes).

The sorter system can sort the tubes after the decapper based on the next stage of the workflow for each of the biological samples in the sample tubes. For example, the sorter system can determine that several sample tubes containing certain biological samples are ordered to run a serologic test, where various tests can be initiated, such as an agglutination assay to determine whether antibodies exposed to certain antigens can cause particle clumping, a precipitation test that can show whether the antigens are similar by measuring for the presence of antibody in body fluids, and a Western blot test that can help identify the presence of antimicrobial antibodies in blood by their reaction with target antigens. These can be used for screening blood and plasma donation samples for infectious diseases to reduce the risk of transfusion transmitted infections in the recipients. Another example of an ordered test that can be assessed for sorting can include immunohematology, which can include a study of antigen-antibody reactions and analogous phenomena related to the pathogenesis and clinical manifestations of blood disorders. Such tests can include blood and/or plasma typing (e.g. a classification of blood based on the presence and absence of antibodies and also based on the presence or absence of inherited antigenic substances on the surface of red blood cells), cross-matching (e.g. testing for compatibility through matching of different blood and/or plasma group systems), and antibody identification (e.g. identification of a protein produced mainly by plasma cells used by the immune system to neutralize pathogens such as bacteria and viruses). Yet another example of an ordered test that can be assessed for sorting can include nucleic acid testing, which is a molecular technique for screening blood donations to reduce the risk of transfusion transmitted infections in the recipients, often used in the same labs as serological tests.

A pooler of the pooler stage 108 can perform the following functions. A pooler system can pool sample tubes together for performing biomedical experiments on the pooled biological samples. Pooling biological specimens can be a cost-efficient sampling strategy. Because two or more specimens can be physically combined into a single pooled unit for analysis, the number of tests may be reduced. The pooling systems can pool several biological components into a pooled set of biological components. Pooling of samples can be used in certain blood and plasma screening labs for indicated Nucleic Acid Testing (NAT) assays. For example, aliquots from multiple samples can be combined into a single pool tube to allow one test (as described in the package inserts of the assay). Pooling information can be supplied to the middleware. If the test is negative, all samples can be deemed negative and released. Otherwise if the pool is positive, the pool can be deconvoluted to resolve which sample is reactive by retesting the samples individually.

The analyzer system of the analyze stage 110 can perform the following functions. Analyzer systems can analyze the biological sample and/or the pooled biological samples. The analyzer system can feed results back to the blood and/or plasma screening laboratory workflow system. When the analyzer systems receive the biological sample and/or the pooled biological samples, the analyzer systems can identify the sample tube(s) and report to the blood and/or plasma screening laboratory workflow system that the biological sample(s) have arrived at the analyzer systems. After analysis, the analyzer systems can report to the blood and/or plasma screening laboratory workflow system that the biological samples(s) are about to leave or have left the analyzer systems. The blood and/or plasma screening laboratory workflow system can then determine the next biological sample to arrive at the analyzer system and determine where the recently analyzed biological sample(s) are to be transported to. The analyzer system can analyze biological samples to determine the presence of abnormalities.

Analyzer systems can identify if the blood and/or plasma carries certain compounds and/or determine the level of compounds in the blood and/or plasma. For example, analyzers can determine the blood sugar levels in the blood. Some examples of analyzers include cardiovascular tests (including a test for total cholesterol, low density lipoprotein, apolipoprotein, lipoprotein, high density lipoprotein, triglycerides, c-reactive, protein, homocysteine, and the like), liver function tests (including a test for alkaline phosphatase, GGT, AST/SGOT, ALT/SGPT, bilirubin, ammonia, and the like), kidney function tests (including a test for creatinine, blood urea nitrogen, and the like), thyroid tests (including a test for TSH, free T3, total T3, free T4, total T4, rT3, and the like), reproductive function tests (including a test for testosterone, IGF-1, growth hormone, DHEA, estradiol, PSA, and the like), carbohydrate tolerance tests (including a test for fasted insulin, fasted glucose, and the like), protein status tests (including a test for albumin, globulin, and the like), vitamin, mineral, and acid/base status tests (including a test for 25-hydrxyvitamin, vitamin b-12, folic acid, calcium, phosphorus, sodium, potassium, chloride, iron, transferrin, total iron binding capacity, ferritin, carbon dioxide, and the like), white blood cell tests (including a test for white blood cells, neutrophils, basophils, eosinophils, lymphocytes, monocytes, and the like), red blood cell tests (including a test for red blood cells, hemoglobin, hematocrit, mean corpuscular volume, mean corpuscular hemoglobin concentration, mean corpuscular hemoglobin, red cell size distribution width, platelets, and the like), and other miscellaneous tests (including a test for cortisol, LDH, uric acid, and the like). In Blood and Plasma Screening Labs, Analyzer systems can identify pathogens including: Nucleic Acid Test: HIV, HBV, HCV, WNV, Zika and Babesia; Immunoassay: HIV, HBV, HCV, HTLV I & II, Chagas, Syphillis and CMV among other tests.

In some embodiments, analyzer systems can measure different chemicals and other characteristics in a plurality of biological samples. Such measurements can occur automatically and/or with minimal human assistance. The process of analyzing the blood and/or plasma samples can include placing test tubes into racks, transporting the tubes along a path, and/or inserting the tubes into circular carousels that rotate to make the sample available.

The transporting between stations (e.g. analyzer systems, pooler systems, etc.) can occur automatically, semi-automatically, manually, and/or a combination thereof. For example, the sample tubes can be transported from the sorter system to the pooler system manually by a technician, and from the pooler system to the analyzer system automatically (e.g. by use of robotics to move the sample tubes). The input of information and data into the software system can also occur automatically, semi-automatically, manually, and/or a combination thereof.

A review system of the review stage 112 can perform the following functions. At a review system, the blood and/or plasma screening laboratory workflow system can receive status from the various systems in the laboratory (e.g. accession system, centrifuge system) about the status and location of the biological samples. The blood and/or plasma screening laboratory workflow system can then determine whether the biological samples are progressing according to the expected workflow. If the biological samples are staying at a particular system for a prolonged period of time, the blood and/or plasma screening laboratory workflow system can readjust the workflow to optimize the workflows of the current and other biological samples. If a system in the laboratory provides an unexpected result of a test to the blood and/or plasma screening laboratory workflow system, the blood and/or plasma screening laboratory workflow system can also readjust the workflow. For example, if the analyzer reports positive (or reactive) results of a particular biological sample which require additional testing of the biological sample, the blood and/or plasma screening laboratory workflow system can readjust the workflows such that the particular biological sample can have the additional testing performed while optimizing the workflow of the other biological samples.

A storage system of the storage stage 114 can perform the following functions. A storage system can receive a biological sample to store in its storage space. This storage space can be storage until the time comes for the following workflow stage, can be a storage space for removal from the laboratory, and/or can be a temporary storage stage before the blood and/or plasma screening laboratory workflow system determines the next step for the biological sample.

### Biological Sample Screening Workflow System

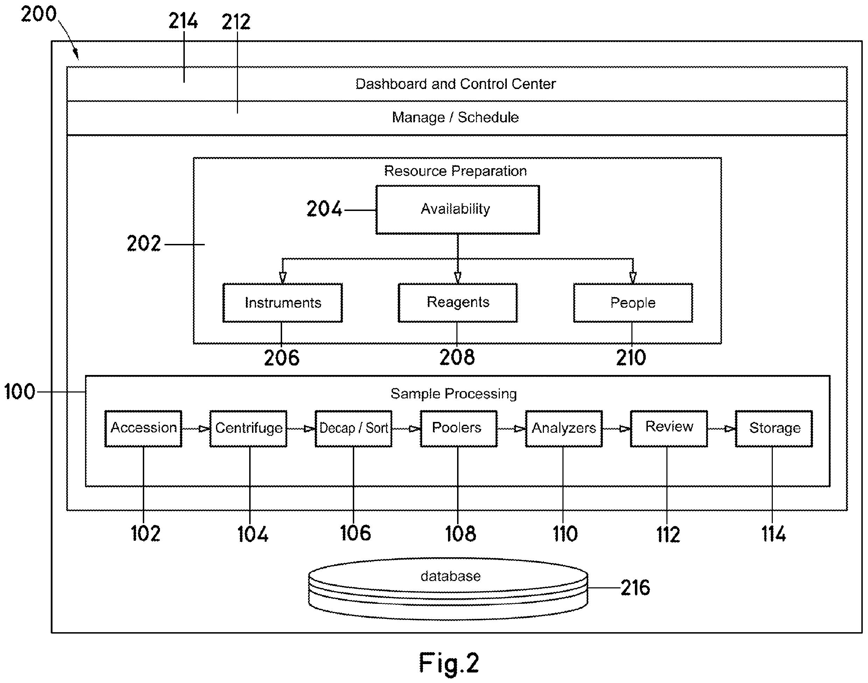

FIG. 2 illustrates a biological sample, such as blood and/or plasma sample, screening workflow system 200 according to some embodiments. The system 200 includes a workflow, such as the workflow 100 of FIG. 1, including the accession stage 102, the centrifuge stage 104, the decap/sort stage 106, the pooler stage 108, the analyzer stage 110, the review stage 112, and the storage stage 114. The system 200 also includes a resource preparation system 202, which includes resource availability 204, resource of instruments 206, resource of reagents 208, and resource of people 210. The system 200 further includes a manage and schedule system 212, a dashboard and control center 214, and a database 216. The screening workflow system 200 can include one or more processors and one or more memories. The one or more processors can control, monitor, or the like one or more biological sample screening devices described herein.

The manage / schedule system 212 of the screening workflow system 200 can determine the workflow for the biological samples. For example, if a large number of urgent biological samples arrive at the laboratory, the manage / schedule system may determine the urgency and quantity of these biological samples via assessing the order, the biological samples, and the like. The manage / schedule system can then prioritize these biological samples to go through the accession system of the accession stage 102, the centrifuge system of the centrifuge stage 104, the decap / sort system of the decap / sort stage 106, the pooler system of the pooler stage 108, the analyzer system of the analyzer stage 110, the review system of the review stage 112, and the storage system of the storage stage 114 before other biological samples that were previously scheduled.

The manage / schedule system 212 can monitor resource availability 204 to determine and/or optimize workflow for the biological samples. The manage / schedule system 212 can assess availability of instruments 206, such as devices for performing tests. For example, if there are more than a sufficient number of instruments to perform steps of the centrifuge stage 104 but a limited supply of instruments to perform steps of the decap / sort stage 106, the workflow may prioritize biological samples that require the decap / sort stage 106 to undergo the centrifuge stage 104 before the biological samples that can bypass the decap / sort stage 106.

The manage / schedule system 212 can assess the availability of reagents 208 to determine and/or optimize workflow for the biological samples. For example, if there are a limited supply of reagents 208 for the analyzers stage 110 for one particular test currently but have data of a new supply of reagents to arrive at the laboratory in 3 hours, the manage / schedule system 212 can initiate workflows for a set number of biological samples such that the limited supply of reagents 208 can be used to assess the set number of biological samples, and place the remaining biological samples into storage 114 until the new supply of reagents arrives. In some embodiments, the manage / schedule system 212 can display to the operator recommendations regarding reagent preparation and loading of reagents on instruments, or removal of reagents from instruments. In some embodiments, the GUI can display to the operator recommendations and/or requirements for workflow changes. In some embodiments, the GUI can allow the operator to make informed choices and proceed with workflows, such as enabling the operator to change the workflow based on efficiency.

The manage / schedule system 212 can monitor the availability of people 210 (for example, technicians) to determine and/or optimize workflow for the biological samples. For example, if the decap / sort stage 106 can be performed manually by technicians at a slower speed and also can be performed automatically by robotics at a faster speed, the manage / schedule system 212 can schedule the workflow such that biological samples that need to be analyzed quickly after direct contact with the environment can be scheduled for automatic decapping and sorting, whereas other biological samples can be directed to technicians for the decap / sort stage 106.

Data related to the instruments, reagents, people, and/or the biological samples can be stored in a database 216, which can be stored in one or more memories. The database 216 can include one or more internal and/or external data sources. In some embodiments, one or more of the data repositories and the data sources described above can be implemented using a relational database, such as DB2, Sybase, Oracle, Code Base, and Microsoft^{®} SQL Server as well as other types of databases such as a flat-file database, an entity relationship database, and object-oriented database, and/or a record-based database.

The systems (for example, the manage / schedule system 212) can access the database 216. The database 216 can be stored in a data repository. The systems can access the database 216 through a network or can directly access the database 216 through I/O devices and interfaces. The database 216 storing the one or more data sources can reside within one of the systems and/or can be external to the systems.

The dashboard and control center 214 can generate graphical user interface data to display to a user, such as a technician. The dashboard and control center 214 can display updated workflow information for one or more biological samples and/or test tubes. The dashboard and control center 214 can display test results of the biological samples. The dashboard and control center 214 can also display availability of instruments, reagents, and people.

### Graphical User interface

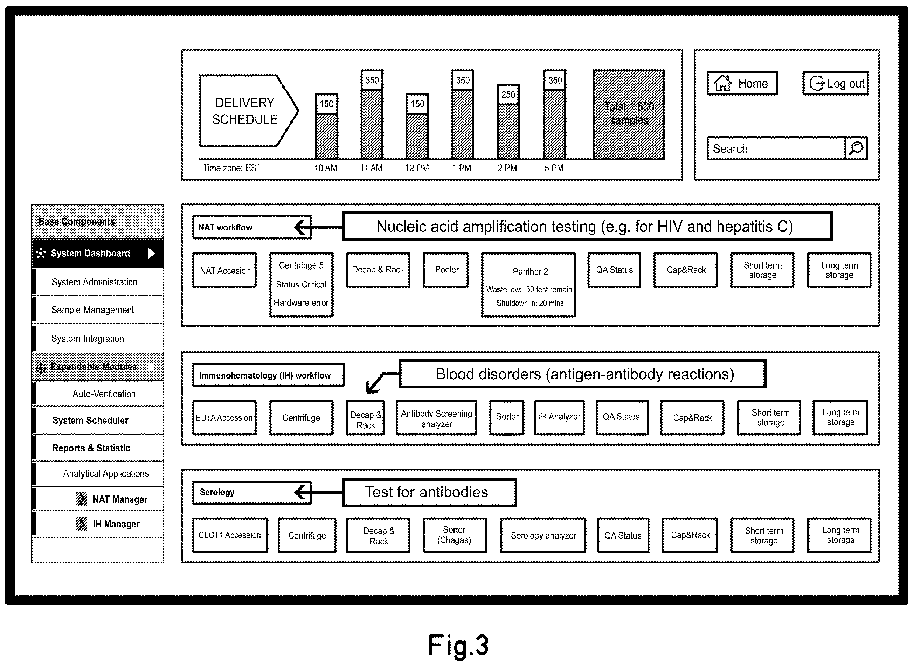

FIG. 3 illustrates an embodiment of a graphical user interface displaying workflows according to some embodiments. As is illustrated, the graphical user interface displays the following workflows: a nucleic acid amplification testing (NAT) workflow, for example for infectious diseases such as HIV, Hepatitis B (HBV), Hepatitis C (HCV), Hepatitis E (HEV), Zika Virus, West Nile Virus (WNV), Parvo, and the like, (NAT workflow), a immunohematology (IH) workflow for antigen-antibody reactions (IH workflow), and a serology workflow for testing antibodies. The graphical user interface can be displayed under control of one or more processors, such as the one or more processors of the screening workflow system 200.

In some embodiments, the NAT workflow can include a NAT accession stage, a centrifuge stage, a decap and rack stage, a pooler stage, an analyzer stage (shown as "Panther 2" in the diagram), a quality assurance (QA) status stage, a cap and rack stage, a short term storage stage, and a long term storage stage. In this example, the centrifuge device #5 is showing a status of critical hardware error. The centrifuge system can identify errors, such as a hardware error, and send the error message to the manage / schedule system. The manage / schedule system can determine from the error message to initiate a fix on the centrifuge device #5. The manage / schedule system can optimize the workflow such that future workflows do not use the centrifuge device #5 but have access to other workflow stages included in the NAT workflow.

The graphical user interface shows that the current status of the analyzer (for example, Panther 2 as illustrated in the diagram) has 50 tests remaining and will shut down in 20 minutes. The manage / schedule system can receive this information from the analyzer system and optimize the workflow to use 50 tests and to avoid sending workflows to the analyzers after 20 minutes.

In some embodiments, the IH workflow includes an EDTA sample tube accession stage, a centrifuge stage, a decap and rack stage, an antibody screening analyzer stage, a sorter stage, an IH analyzer stage, a QA status stage, a cap and rack stage, a short term storage stage, and a long term storage stage.

In some embodiments, the serology workflow includes a CLOT sample tube accession stage, a centrifuge stage, a decap and rack stage, a sorter stage, a serology analyzer stage, a QA status stage, a cap and rack stage, a short term storage stage, and a long term storage stage.

The graphical user interface can include a delivery schedule for samples to arrive at the laboratory. The delivery schedule can include a total number of samples for the day (for example, 1,600 total samples), and a number of samples at certain time periods (for example, 150 at 10AM, 350 at 11AM, 150 at 12PM, 350 at 1PM, 250 at 2PM, and 350 at 5PM).

The manage / schedule system can track the progress of analysis of biological samples and generate progress indications via a graphical user interface.

In some embodiments, the manage / schedule system can identify a device, a biological sample, a reagent, and/or a technician of interest. The device, a biological sample, a reagent, and/or a technician of interest can be identified via a user input and/or automatically based on criteria set by the manage / schedule system, as described in further detail below. The manage / schedule system can determine the progress of the device, a biological sample, a reagent, and/or a technician of interest, and display a progress indicator via a graphical user interface.

### Managing and Scheduling Workflow

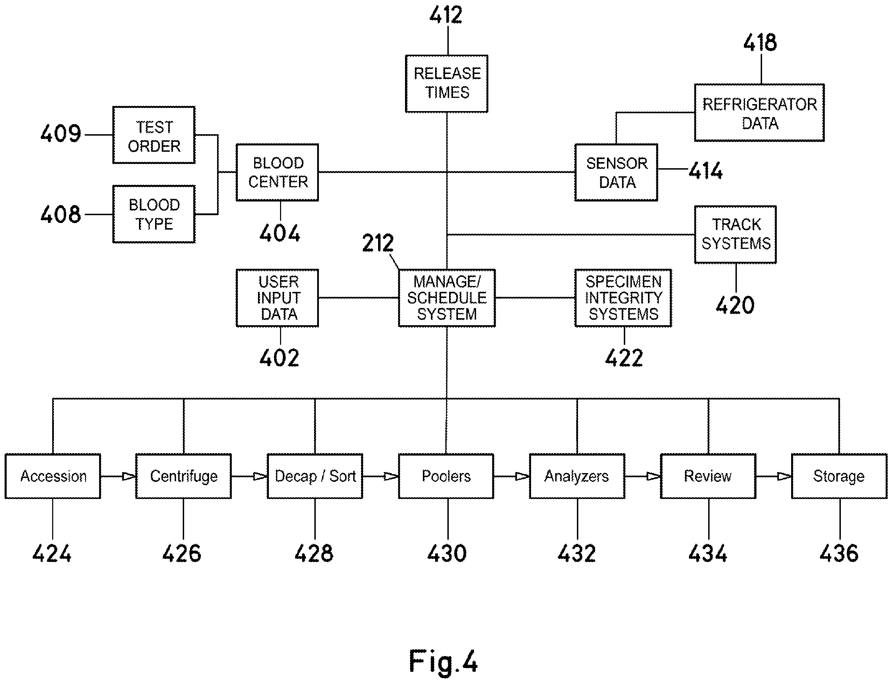

FIG. 4 illustrates data used for managing and scheduling workflow according to some embodiments. A manage / schedule system, such as the system 212, can monitor the operation of a variety of systems to generate a warning or failure indication via a graphical user interface. The manage / schedule system 212 can store the tracked progress into a database. The manage / schedule system 212 can acquire information from a variety of computing systems in order to prevent errors, run necessary tests, inform operators of status, direct operators to perform certain actions, prepare documentation, run follow-up tests (such as reflex/retest), and/or assist reviewers for making conclusions and forms from results.

The manage / schedule system 212 can receive input from various systems. The systems can be located throughout the laboratory. The manage / schedule system 212 can receive input from systems associated with the stages of the workflow shown in FIG. 1, such as an accession system 424, a centrifuge system 426, a decap / sort system 428, a pooler system 430, an analyzer system 432, a review system 434, and a storage system 436.

The manage / schedule system 212 can receive user input data 402. For example, the manage / schedule system 212 can receive input from a technician. The input may be data that can affect a workflow. For example, the technician can input information on a hardware or software error of a system, a result of a test run by an analyzer, status that the biological sample is placed or removed from a system, and the like. Then, the manage / schedule system 212 can optimize the workflow based on the user input.

The manage / schedule system 212 can receive information from the blood and/or plasma center 404. The blood and/or plasma center can include information such as the blood and/or plasma type 408 of the sample, the test order 409, and the like. The manage / schedule system 212 can receive release times of the biological samples 412.

The manage / schedule system 212 can receive sensor data 414. The sensor data can come from a system of the blood and/or plasma screening and diagnostic laboratory.

The manage / schedule system 212 can receive data from track systems 420. The track systems 420 can perform tracking of data for workflow management. For example, the tracking systems 420 can track inventory, reagents, technicians, and/or biological samples throughout the laboratory.

The manage / schedule system 212 can receive specimen integrity data from specimen integrity systems 422. The specimen integrity systems 422 can test the integrity of the specimen in particular phases of the workflow, before the biological sample is placed into a workflow, during and/or after the biological sample completed a workflow. The specimen integrity system 422 can check the specimen manually and/or automatically. For example, a sorter with this function can check for sufficient volume, lipemia and hemolysis can be checked based on color of the specimen after centrifugation.

For example, the manage / schedule system 212 can determine not to send a test order to an analyzer if specimen integrity checks fail. The manage / schedule system 212 can prevent such a release until the error command is lifted from the analyzer system and a user command is provided to allow such release.

The manage / schedule system 212 can receive data from the systems in FIG. 4 for multiple workflows and evaluate the received data in multiple workflows to determine the state of the biological sample. The manage / schedule system 212 can also evaluate this information to determine the state of the various systems in the laboratory. The state of the biological samples and the systems, and the various workflows can then be displayed on a graphical user interface. The technicians can then view real time status of the current operating state for each element in the workflow and the overall operating state of the workflow without searching for biological samples or observe performance on a particular device.

### Managing Test Orders

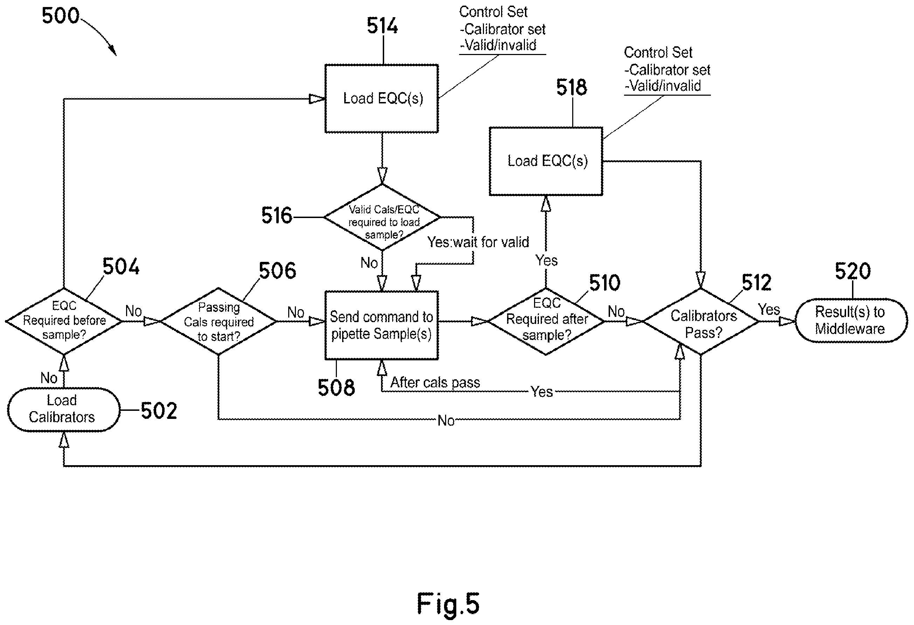

FIG. 5 illustrates an embodiment of a process 500 for managing test orders according to some embodiments. The process 500 can be implemented by a screening workflow system, such as the system 200. In some implementations, the process 500 can be implemented by a manage / schedule system, such as the system 212. The process 500 can allow configuration of external controls. The results of the configuration of external controls can impact the release of sample test conclusions over a period of time and/or over a number of tests. The affected tests can be performed before and/or after external controls are performed. The configuration of external controls can match requirements for laboratory procedures. At block 502, the process 500 loads the calibrators. Calibrators can include manufactured samples containing known expected results when analyzed by the analyzers. For example, the Procleix Ultrio Elite can include 6 positive calibrator replicates and 3 negative calibrator replicates. EQCs can include external quality controls with known expected results, and can be purchased by testing labs from third parties. The EQCs can run in frequency associated with a number of tests, an amount of time, and/or a combination of both.

At block 504, the process 500 determines whether configuration of external controls are required before loading the sample. If the configuration of external controls is required before testing a sample, then the process continues to block 514 where the configuration of external controls is loaded. The control set can include a calibrator set and/or a flag indicating whether the control set is valid or invalid. A valid calibrator or control can produce expected results when run through the analyzers. For example, for qualitative tests, a positive calibrator/control can indicate reactive state for the test, and a negative calibrator/control can indicate a non-reactive state. Quantitative calibrators and controls can be valid when their results are in an expected pre-defined range.

At block 516, the process 500 determines whether the valid calibrators and/or external controls are required to be completed before the sample is loaded. If so, then the process 500 waits for a valid configuration of these calibrators and/or controls, and continues to block 508. If the process 500 does not require valid calibrators and/or configuration of external controls, then the process continues to block 508 without waiting for a valid calibrator and/or configuration of external controls.

At block 508, a command is sent to the Analyzer to pipette the sample. The sample may have been manually loaded or automatically transported to the Analyzer to be ready to be pipetted.

At block 504, if a configuration of external controls is not required before loading the sample, the process continues to block 506, where the process 500 determines whether passing calibrators are required to start loading and testing of the samples. If so, then the process continues to block 512 where the process 500 determines whether the calibration passes. If calibration results are not required to start testing samples, the process continues to block 508 where a command is sent to the instrument to start testing the sample.

At block 510, the process 500 determines whether a configuration requires loading of external controls after sample loading. If so, then the process continues to block 518 where the configuration of external controls is loaded. The configuration of external controls loaded in block 518 can include similar configuration of external controls loaded in block 514. The timing to load the EQCs can be defined by individual labs, and the samples can be loaded as a group for the various tests running on an analyzer. After loading the configuration of external controls, the process can continue to block 512 where the process 500 determines whether the calibrators pass. Calibrators can run as if they are samples to be tested on the instruments. The instruments can identify when calibrators are loaded, and/or instruments can label the results as from calibrators accordingly. Information regarding whether calibrators pass (for example, result as expected for the calibrator type) or fail can be communicated by the instrument to the middleware.

At block 512, if the calibrators pass, then the process can proceed to transmitting results the middleware. Assay test results (sample reactive, sample non-reactive, calibrator valid/invalid, EQC valid/invalid can be passed to the middleware. If the calibrators do not pass, then the process returns to block 502. In some embodiments, if calibrators do not pass, results using that reagent kit on that instrument are considered valid, and valid calibrators results can be requested to obtain valid sample results.

### Managing Release of Test Results

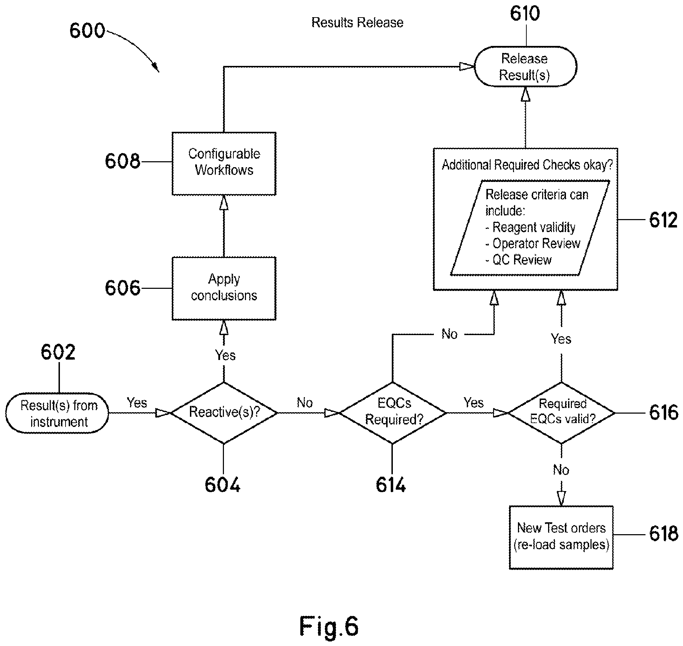

FIG. 6 illustrates an embodiment of a process 600 for managing the release of test results according to some embodiments. The process 600 can be implemented by a screening workflow system, such as the system 200. In some implementations, the process 600 can be implemented by a manage / schedule system, such as the system 212. The process 600 can order the release of test results. The release of the test results can, in some circumstances, be based on the configuration of external controls. A configuration of external controls may prevent the release of certain biological samples while allowing the release of other biological samples. In some embodiments, the external controls can be loaded into an analyzer.

At block 602, results are received from an instrument, such as the results of testing on a biological sample by an analyzer system. The results are assessed to determine whether they are reactive at block 604. A reactive test result can include a positive sample for the test (for example, infected with the pathogen). A reactive pool can mean at least one constituent in the pool is infected.

If the results are reactive, then the conclusions are applied to the test for that sample at block 606, and the workflows are configured accordingly at block 608. Conclusions can include intermediate or final results that indicates if the sample is infected, the provided calibrator and EQC results can be valid. The workflows can be configured by the lab. For example, confirmation tests can be required when a sample is initially found reactive. In another example, workflows can include how many positive retest results would confirm a final positive result. At block 610, the process 600 can release the results from the instruments. The results can be released to the lab information system (LIS), to the GUI for the technicians to view results, and/or to the next stage of the workflow.

At block 614, the process 600 can determine whether configuration of external controls are required. The sequence for EQC can depend on the protocol set by the laboratory and/or can be before determining reactivity and/or after determining reactivity. If configuration of external controls are not required, then at block 612, the process 600 can perform additional checks if available. For example, the process 600 can check whether the reagent is valid, whether quality control review and/or operator review is valid, and the like. Then, the process 600 can release the results from the instruments.

At block 614, if configuration of external controls are required, then the process 600 can determine whether the tested external controls are valid. If they are valid, then the process can continue to block 612 where additional checks can be performed and at block 610, the test results released. At block 616, if the external controls are not valid, then at block 618, new test orders are requested (for example, reloading biological samples). In some embodiments, if calibrator or EQC result is invalid, the test result may not be trusted.

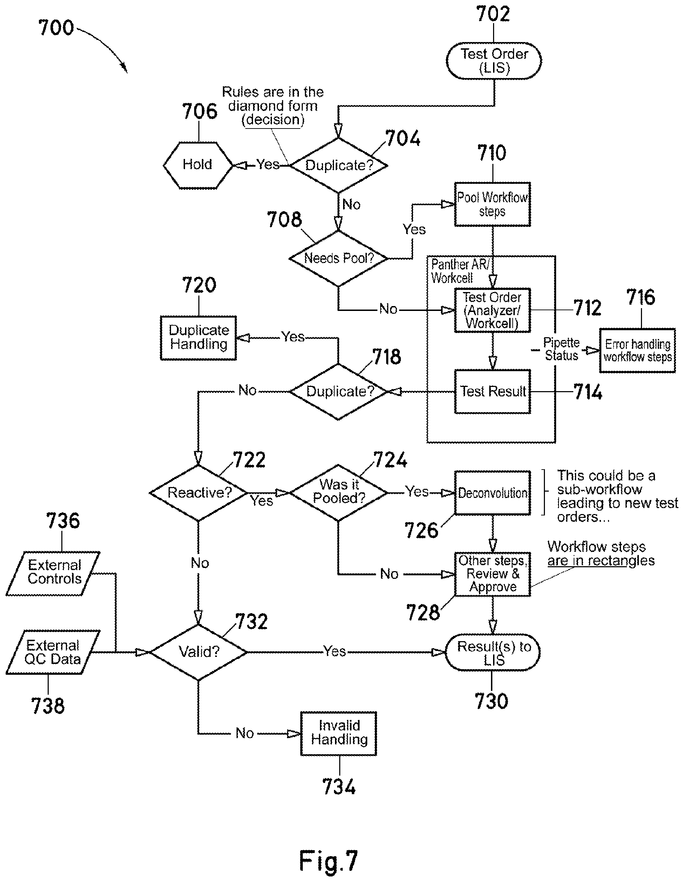

FIG. 7 illustrates a process 700 for evaluating potential constituents of a pool to prevent unwanted pools or tests according to some embodiments. The process 700 can be implemented by a screening workflow system, such as the system 200. In some implementations, the process 700 can be implemented by a manage / schedule system, such as the system 212. An unwanted pool can comprise constituents that may already be a part of another pool scheduled for the same test, or constituents that do not have the same pooling requirements as other biological samples in the pool. For example, a potential pool can have constituents that do not all have the same pooling requirements because some constituents are to be pooled for two assays whereas the remaining constituents are to be pooled for a single assay.

The process 700 can determine a default workflow comprising of workflow steps, rules, and procedures. FIG. 7 illustrates an example of a workflow that includes pooling steps if pools are needed, and if so will also check for valid pool results. At block 702, the process 700 can receive an order for a test from a Laboratory Information System (LIS).

At block 704, the process 700 can check whether the test order is a duplicate. The duplicate can include an unwanted pool and/or test, and vice versa. The duplicate can include confirmation test rerun (for example, because the initial test was reactive). The unwanted duplicate can already be part of another pool and/or already tested individually.

If the process 700 determines that the test order is a duplicate, then the test order is placed on hold at block 706. One advantage is that the workflow for the test order does not have to be performed, and as such, time and resources of the laboratory are saved. Furthermore, results for the test order can be reported if the duplicate test order was already performed previously. The technician can manually determine what to do with the sample based on laboratory procedure.

If the process 700 determines that the test order is not a duplicate, the process can proceed to block 708.

At block 708, the process 700 can determine whether the biological sample of the test order can be pooled with other biological samples for testing. If the process 700 determines that the biological sample can be pooled with other samples, the process can send the biological sample to the pooling system and initiate the pool workflow procedures at block 710. Then at block 712, the test order can be performed and the biological sample analyzed. A workcell can include a group of analyzers connected to an automation system. If the process 700 determines that the biological sample does not need to be pooled, then the test order can be performed and the biological sample analyzed at block 712. Test results can be generated at block 714 based on the performance of the test order at block 712. The Panther AR/Workcell analyzer can be used to perform the test order and generate the test result. The analyzer can include an analyzer that can be connected to an automation system.

The workcell can transport the biological sample to the appropriate instruments to perform the test order. Based on a pipette status, error handling workflow steps can be generated at block 716.

At block 718, the process 700 can determine whether the test order was a duplicate. In some embodiments, more resolution options are available if the duplicate is detected before testing than after testing. If the process 700 determines that a duplicate exists, the process transitions to 720 where it handles the duplicate. The laboratory can define a procedure to continue with the test and/or redo the pool depending on the identified duplicate. In some embodiments, duplicates can be required to get a valid result or for confirmation testing. In some embodiments, duplicate testing can include documentation explaining why duplicate test events occur.

Otherwise, the process 700 transitions to block 722 where the test result is assessed for reactivity. If the test results are reactive, then at block 724, the process 700 determines whether the biological samples were pooled. If the biological samples were pooled, then at block 726, the process 700 can deconvolute the test orders to each individual test order and/or biological sample, and at block 728, further steps can be performed such as reviewing and approving the test results. In some embodiments, the steps can include confirmation tests and/or discriminatory tests for multiplex assays. If the process 700 determines that the biological samples were not pooled, then further steps can be performed in block 728. At block 730, the results of the test can be transmitted to the LIS.

At block 722, if the test results are not reactive, then the test results are checked for validity. Block 722 can include results from analyzers. Non-reactive test results from analyzers can require controls tests to be valid for final results to be valid. Configuration of external controls at block 736 and external quality control data at block 738 can be used to determine the validity of the test results. If the test results are valid, then the test results can be transmitted to the LIS in block 730. If the test results are not valid, then a message can be sent to the process 700 that the test was improperly handled and that the test results are invalid.

### Pool Validity

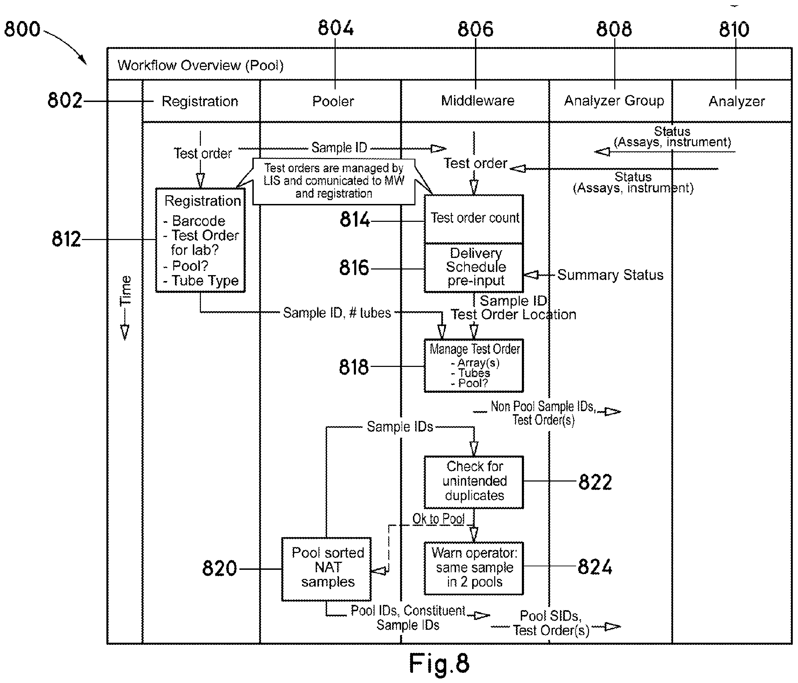

FIG. 8 illustrates a process 800 for determining if constituents in a pool are valid before testing according to some embodiments. The process 800 can be implemented by a screening laboratory system, which can include a registration system 802, a pooler system 804, a middleware system 806, an analyzer group system 808, and an analyzer system 810. In some implementations, the middleware system 806 is similar to a manage / schedule system, such as the system 212.

At the registration system 802, a biological sample ID can be assigned to the biological sample by the process 800, and passed to other systems such as the middleware system 806. At the analyzer system 810, the status of the analyzer and other systems can be generated and transmitted to other systems, such as the middleware and/or the analyzer group systems.

At the middleware system 806, a test order can be received 814 from the LIS, and at block 816, the middleware system 806 can receive a delivery schedule. The delivery schedule can comprise an estimate of the delivery times to the lab of batches of donor samples from donor centers. The delivery schedule can be pre-input. The delivery schedule can be automatically sent from the donor centers or may be manually input in a previous workflow step by laboratory personnel. The middleware may also estimate the times batches of test results are expected to be available and delivered to clients. The delivery schedule can be used to optimize scheduling of samples and tests before new samples are loaded onto the input module of the Analyzer Group 808 (Workcell), in order to meet the results release expectations of clients. The results release schedule can be calculated based on tests yet to be done, and/or how many and/or what tests are currently running on the analyzers. The analyzer group system 808 can generate a summary status, and transmit the summary to the middleware system 806 to be used with the sample delivery schedule 816 at the middleware system 806 to manage sample testing and optimize Turn Around Time (TAT) for the lab clients.

The registration system 802 can register the test order 812. The registration can occur via a barcode on a sample tube. The registration can identify characteristics of a test order and/or a biological sample, such as the tube type or whether the sample is to be pooled. The registration system 802 can transmit information to other systems. For example, the registration system 802 can transmit the sample ID and number of tubes to the middleware system 806.

The middleware system 806 can manage the received test order 818. For example, the test order can be managed based on the assay, the tube, and/or whether the biological sample is to be pooled for each test. The determination of whether or not a test can be pooled can be dependent on the assay (test type) and/or when the last reactive test has been found at that donation location. The non-pool sample IDs and/or the test orders can be transmitted by the middleware system 806 to the analyzer group system 808. Some laboratories may not include pooling and/or pool a subset of tests. Some NAT tests can be pooled. A sample from one donor can undergo multiple different tests. In some embodiments, a subset of components are pooled. In some embodiments, the biological components are not pooled.

The middleware system 806 can check whether the samples are unintended duplicates. For example, the samples may already be a part of another pool scheduled for the same test, or samples that do not have the same pooling requirements as other biological samples in the pool. If the samples are ok to pool, then the middleware system 806 may send a command to the pooler system 804 to pool the sorted NAT samples. The pool IDs and the constituent sample IDs can then be transmitted from the pooler system 804 to the middleware system 806. Otherwise, the middleware system 806 may warn the operator (for example, that the same sample is in two pools) 824. The Pool IDs and the test orders can be sent from the middleware system 806 to the analyzer group 808 for performing analysis on the pooled samples.

### Reactive Pools

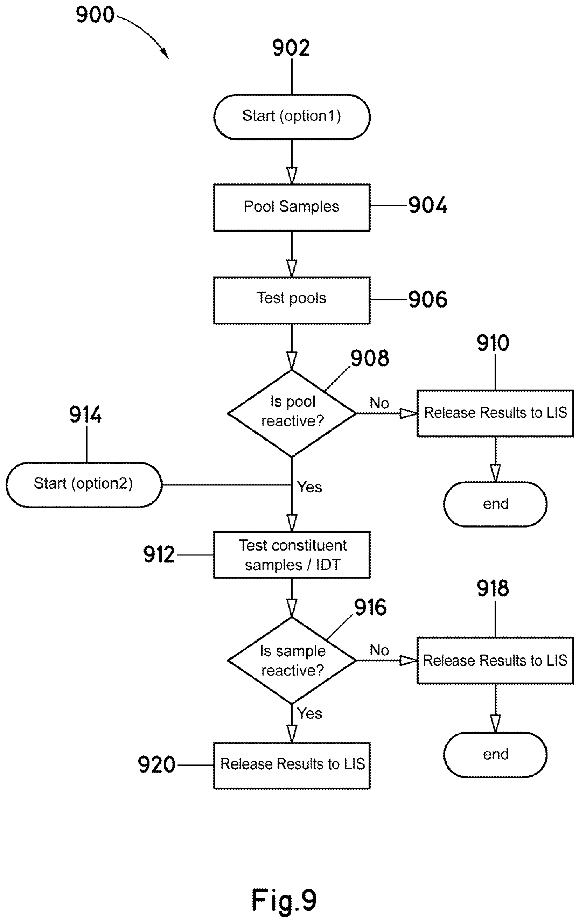

FIG. 9 illustrates a process 900 for deconvoluting a reactive pool according to some embodiments. The process 900 can be implemented by a screening workflow system, such as the system 200 or the system illustrated in FIG. 8. In some implementations, the process 900 can be implemented by the middleware 806 or a manage / schedule system, such as the system 212. Deconvolution in this context can indicate the one or more individual sample(s) that caused the pool to be tested reactive can be identified by testing each constituent sample.

The process 900 can begin at block 902, and at block 904, the biological samples can be pooled. At block 906, the biological samples are tested. At block 908, the process 900 can determine whether the pool is reactive. A reactive pool can have constituents run on one of several analyzers. If the process 900 determines the pool is not reactive, the results can be released to LIS at block 910, and the process can end.

If the process 900 determines that the pool is reactive, then at block 912, the constituent samples can be tested. Each individual biological sample can be tested, or in other words, individual donation testing (IDT) can occur. If pooling is not required, then the process can start at block 914 and continue to block 912.

At block 916, the process 900 tests whether each sample is reactive. If the sample is or is not reactive, the results can be released to LIS at block 918 or 920. In some embodiments, the results of the samples in the pool can be released to LIS even though the pool is reactive. In some embodiments, the samples can be released to the LIS whether or not the pool is reactive because they are valid results. In some embodiments, the lab can identify if the blood, plasma, and/or organ donations can be used. In some embodiments, others steps can be taken after block 916 if the sample is reactive after 920 (such as retesting and/or running discriminatory tests for multiplexed assays).

### Display List of Actions for Operator

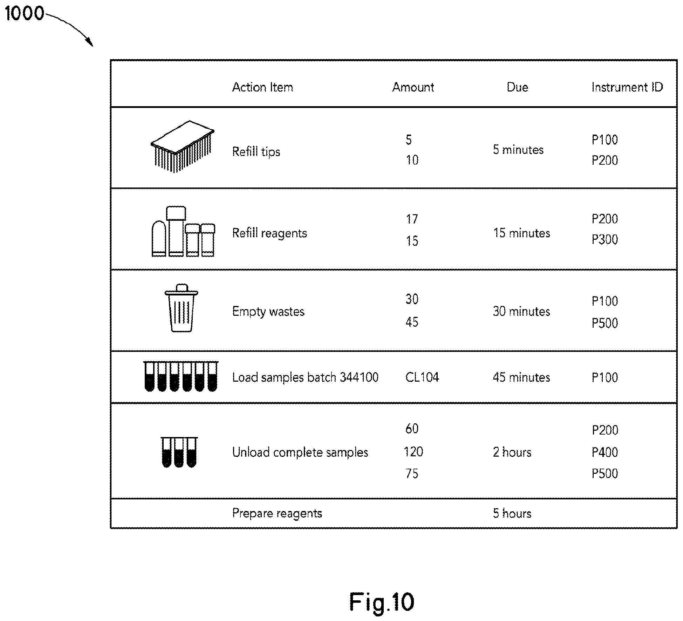

FIG. 10 illustrates a display 1000 of a list of actions for an operator to ensure efficient operation of the system and release of results according to some embodiments. In some embodiments, a list of actions can be displayed for operators including information useful for the operators. In some embodiments, the displayed list of actions can include one or more action items, such as refill tips at one or more instruments, refill reagents, empty waste, load sample batch(es), unload completed samples, prepare reagents, or the like. In some embodiments, the graphical user interface displays a representative picture of one or more action items.

In some embodiments, the displayed list of actions can include an estimated amount of time by when a particular action is to be performed. In some embodiments, the amount of time can be displayed in increments of seconds, minutes, hours, periods of time, morning, afternoon, evening, a day of the week, a date, and the like. In some embodiments, the amount of items can be displayed. For example, 5 refill tips can be suggested and/or required for instrument ID P100, and 10 refill tips can be suggested and/or required for instrument P200.

In some embodiments, the displayed list of actions can include an identifier for an instrument, such as an instrument identifier. As illustrated in 1000, the action items can be sorted based on a measurement of urgency (for example, the amount of time remaining before one or more actions should be taken). In some embodiments, the action items can be sorted based on an instrument identifier, in alphabetical order, and the like. The list of items can be grouped by urgency, action item, instrument ID, and the like (the example 1000 shows grouping by action item, sorted by urgency).

### Other Variations

Although the foregoing describes some embodiments related to blood and/or plasma screening, the disclosed systems and methods are applicable to screening of biological samples other than blood and/or plasma. Such biological samples can include urine, tissue, cells, or the like. Any value of a threshold, limit, duration, etc. provided herein is not intended to be absolute and, thereby, can be approximate. In addition, any threshold, limit, duration, etc. provided herein can be fixed or varied either automatically or by a user. Furthermore, as is used herein relative terminology such as exceeds, greater than, less than, etc. in relation to a reference value is intended to also encompass being equal to the reference value. For example, exceeding a reference value that is positive can encompass being equal to or greater than the reference value. In addition, as is used herein relative terminology such as exceeds, greater than, less than, etc. in relation to a reference value is intended to also encompass an inverse of the disclosed relationship, such as below, less than, greater than, etc. in relations to the reference value. Moreover, although blocks of the various processes may be described in terms of determining whether a value meets or does not meet a particular threshold, the blocks can be similarly understood, for example, in terms of a value (i) being below or above a threshold or (ii) satisfying or not satisfying a threshold.

While certain embodiments have been described, these embodiments have been presented by way of example only, and are not intended to limit the scope of protection.

Conditional language, such as "can," "could," "might," or "may," unless specifically stated otherwise, or otherwise understood within the context as used, is generally intended to convey that certain embodiments include, while other embodiments do not include, certain features, elements, or steps. Thus, such conditional language is not generally intended to imply that features, elements, or steps are in any way required for one or more embodiments or that one or more embodiments necessarily include logic for deciding, with or without user input or prompting, whether these features, elements, or steps are included or are to be performed in any particular embodiment. The terms "comprising," "including," "having," and the like are synonymous and are used inclusively, in an openended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, or 0.1 degree.

The scope of the present invention is not intended to be limited by the specific disclosures of preferred embodiments in this section or elsewhere in this specification, and is defined by claims as presented in this section or elsewhere in this specification or as presented in the future.

## Claims

1. A system (200) for analyzing biological samples, the system comprising:
a plurality of centrifuges (426) configured to separate at least some biological samples from a plurality of biological samples into a plurality of components;
a plurality of pooling devices (430) adapted so as to pool a plurality of subsets of the plurality of components into a plurality of pooled biological components;
a plurality of biological sample analyzing devices (432) configured to analyze the plurality of biological samples and/or the plurality of pooled biological components to determine presence of one or more abnormalities;
a processor adapted so as to communicate with and control the plurality of centrifuges (426), plurality of pooling devices (430), and plurality of biological sample analyzing devices (432), the processor further adapted so as to:
monitor operation of the plurality of centrifuges (426), plurality of pooling devices (430), and plurality of biological sample analyzing devices (432) and generate at least one of a warning or failure indication via a graphical user interface (214);
track progress of analysis of the plurality of biological samples and generate a plurality of progress indications via the graphical user interface (214); and
store at least some of the tracked progress in memory,
wherein the processor is further configured to:
determine whether one or more of the operations of the plurality of pooling devices (430) and plurality of biological sample analyzing devices (432) are performed on biological samples that are unintended duplicates;
in response to determining that the one or more of the operations of the plurality of pooling devices (430) and plurality of biological sample analyzing devices (432) are performed on biological samples that are unintended duplicates, automatically change the workflow for the one or more of the operations of the plurality of centrifuges (426), plurality of pooling devices (430), and plurality of biological sample analyzing devices (432) that are performed on biological samples that are unintended duplicates,
wherein the duplicates are one or more biological samples that are already a part of another pool scheduled for the same biological sample analyzing device (432), or wherein the duplicates include one or more biological samples that do not have the same pooling requirement as another biological sample in the same pool.

2. The system of claim 1, wherein the plurality of biological samples comprise blood and/or plasma samples.

3. The system of one or more preceding claims, wherein the plurality of biological sample analyzing devices are configured to perform at least one of nucleic acid amplification testing (NAT), immunohematology, or serology.

4. The system of one or more preceding claims, wherein the processor is further configured to, in response to receiving a search request associated with a particular biological sample, determine progress of analysis of the particular sample and generate a progress indication via the graphical user interface (214).

5. The system of one or more preceding claims, wherein the processor is further configured to:
determine presence of a first external quality control preventing a release of a biological component to a biological sample analyzing device (432); and
in response to the determination that the first external quality control is present, prevent release of biological components to the biological sample analyzing device (432) to perform analysis of the biological components until the first external quality control is released.

6. The system of claim 5, wherein the processor is further configured to:
determine presence of a second external quality control preventing a release of the biological components after analysis by the biological sample analyzing device (432); and
in response to the determination that the second external quality control is present, prevent release of an analysis result for the biological components until the second external quality control is released.

7. The system of one or more preceding claims, wherein the processor is further configured to:
in response to a determination that a potential pool includes the first component of the first biological sample configured to be analyzed with multiple assays by one or more biological sample analyzing devices (432) and the second component of the second biological sample configured to be analyzed with the single assay by one or more biological sample analyzing devices (432), prevent the first or second component from being added to the pool.

8. The system of one or more preceding claims, wherein the processor is further configured to:
in response to a determination that a pool is reactive, provide the pool to the same or another biological sample analyzing device after a first biological sample analyzing device (432) completes analysis of the pool, and/or
in response to a determination that a pool is reactive, deconvolve the pool by providing the constituents of that pool to the same or another biological sample analyzing device (432) after the first biological sample analyzing device (432) completes analysis of the pool, and/or
in response to a determination that a constituent in the pool is reactive, provide the constituent to the same or another biological sample analyzing device (432) after analysis of the constituent of the pool is completed.

9. The system of one or more preceding claims, wherein the biological samples are transported among the plurality of centrifuges (426), the plurality of pooling devices (430), and the plurality of biological sample analyzing devices (432) automatically via track system.

10. The system of one or more preceding claims, wherein the processor is further configured to:
automatically adjust a workflow of one or more of the plurality of biological samples based on an unexpected result of one or more of the monitored operation of the plurality of centrifuges (426), the plurality of pooling devices (430), or the plurality of biological sample analyzing devices (432), or the tracked progress of analysis of the plurality of biological samples.

11. The system of claim 10, wherein the processor is further configured to automatically adjust the workflow based on a delivery schedule of biological samples.

12. The system of claim 10, wherein the processor is further configured to automatically adjust the workflow based on one or more of an availability of the plurality of centrifuges (426), the plurality of pooling devices (430), the plurality of biological sample analyzing devices (432), a plurality of reagents, or a plurality of technicians.

13. The system of one or more preceding claims, wherein the processor is further configured to:
receive an error message from one or more of the plurality of centrifuges (426), the plurality of pooling devices (430), or the plurality of biological sample analyzing devices (432); and
generate an error message indication via the graphical user interface (214), wherein the error message includes one or more of a hardware error or a software error and wherein the workflow is automatically adjusted further based on the error message.

14. A method for analyzing biological samples in a system according to any of claims 1 to 13, the method comprising the steps of:
separating, by the plurality of centrifuges (426), at least some biological samples from the plurality of biological samples into a plurality of components,
pooling, by the plurality of pooling devices (430), a plurality of subsets of the plurality of components into a plurality of pooled biological components, and
analyzing, by the plurality of biological sample analyzing devices (432), the plurality of biological samples and/or the plurality of pooled biological components to determine presence of one or more abnormalities
wherein the processor is adapted to further:
communicate with and control a plurality of centrifuges (426), a plurality of pooling devices (430), and a plurality of biological sample analyzing devices (432);
monitor operation of the plurality of centrifuges (426), plurality of pooling devices (430), and plurality of biological sample analyzing devices (432) and generate at least one of a warning or failure indication via a graphical user interface (214);
track progress of analysis of a plurality of biological samples and generate a plurality of progress indications via the graphical user interface (214); and
store at least some of the tracked progress in memory,
wherein the processor is further configured to:
determine whether one or more of the operations of the plurality of pooling devices (430) and plurality of biological sample analyzing devices (432) are performed on biological samples that are unintended duplicates;
in response to determining that the one or more of the operations of the plurality of pooling devices (430) and plurality of biological sample analyzing devices (432) are performed on biological samples that are unintended duplicates, automatically change the workflow for the one or more of the operations of the plurality of centrifuges (426), plurality of pooling devices (430), and plurality of biological sample analyzing devices (432) that are performed on biological samples that are unintended duplicates,
wherein the duplicates are one or more biological samples that are already a part of another pool scheduled for the same biological sample analyzing device (432), or wherein the duplicates include one or more biological samples that do not have the same pooling requirement as another biological sample in the same pool.

15. The method of one or more preceding claims, wherein the method further comprises in response to receiving a search request associated with a particular biological sample, determining progress of analysis of the particular biological sample and generating a progress indication via the graphical user interface (214).

16. A method of one or more preceding claims, wherein the method further comprises:
displaying a list of items, the list of items one or more of comprising:
one or more actions for an operator, said one or more actions comprising: refill tips at one or more instruments, refill reagents, empty waste, load sample batch(es), unload completed samples, or prepare reagents;
an amount of time for the action; or
an instrument identifier,
wherein the list of items is sorted by one or more measures of urgency of the associated actions.

17. The method of one or more preceding claims, wherein the list of items comprises a unique identifier for one or more instruments associated with actions of the same type and urgency.

## Patentansprüche

1. System (200) zum Analysieren für biologische Proben, wobei das System Folgendes umfasst:
eine Vielzahl von Zentrifugen (426), die dazu konfiguriert sind, mindestens einige biologische Proben aus einer Vielzahl biologischer Proben in eine Vielzahl von Komponenten zu trennen;
eine Vielzahl von Pooling-Vorrichtungen (430), die dazu dafür angepasst sind, eine Vielzahl von Teilmengen der Vielzahl von Komponenten in eine Vielzahl gepoolter biologischer Komponenten zu poolen;
eine Vielzahl von Analysevorrichtungen (432) für biologische Proben, die dazu konfiguriert sind, die Vielzahl biologischer Proben und/oder die Vielzahl gepoolter biologischer Komponenten zu analysieren, um das Vorhandensein einer oder mehrerer Anomalien zu bestimmen;
einen Prozessor, der dazu angepasst ist, mit der Vielzahl von Zentrifugen (426), Vielzahl von Pooling-Vorrichtungen (430) und Vielzahl von Analysevorrichtungen (432) für biologische Proben in Kommunikation zu stehen und diese zu steuern, wobei der Prozessor ferner angepasst ist zum:
Überwachen des Betriebs der Vielzahl von Zentrifugen (426), Vielzahl von Pooling-Vorrichtungen (430) und Vielzahl von Analysevorrichtungen (432) für biologische Proben und Erzeugen mindestens einer Warnung oder einer Störungsangabe über eine grafische Benutzeroberfläche (214);
Verfolgen des Fortschritts der Analyse der Vielzahl biologischer Proben und Erzeugen einer Vielzahl von Fortschrittsangaben über die grafische Benutzeroberfläche (214); und
Speichern mindestens einiger der verfolgten Fortschritte in dem Speicher,
wobei der Prozessor ferner konfiguriert ist zum:
Bestimmen, ob einer oder mehrere der Vorgänge der Vielzahl von Pooling-Vorrichtungen (430) und der Vielzahl von Analysevorrichtungen (432) für biologische Proben an biologischen Proben ausgeführt werden, die unbeabsichtigte Duplikate sind;
als Reaktion auf das Bestimmen, dass der eine oder die mehreren der Vorgänge der Vielzahl von Pooling-Vorrichtungen (430) und der Vielzahl von Analysevorrichtungen (432) für biologische Proben an biologischen Proben durchgeführt werden, die unbeabsichtigte Duplikate sind, den Arbeitsablauf des einen oder der mehreren Vorgänge der Vielzahl von Zentrifugen (426), Vielzahl von Pooling-Vorrichtungen (430) und Vielzahl von Analysevorrichtungen (432) für biologische Proben, die an biologischen Proben durchgeführt werden, automatisch zu ändern,
wobei die Duplikate eine oder mehrere biologische Proben sind, die bereits Teil eines anderen Pools sind, der für dieselbe Analysevorrichtung (432) für biologische Proben geplant ist, oder wobei die Duplikate eine oder mehrere biologische Proben beinhalten, die nicht dieselbe Pooling-Anforderung wie eine andere biologische Probe in demselben Pool aufweisen.

2. System nach Anspruch 1, wobei die Vielzahl biologischer Proben Blut- und/oder Plasmaproben umfassen.

3. System nach einem oder mehreren vorstehenden Ansprüche, wobei die Vielzahl von Analysevorrichtungen für biologische Proben dazu konfiguriert sind, mindestens eines von Nukleinsäure-Amplifikationstesten (NAT), Immunhämatologie oder Serologie durchzuführen.

4. System nach einem oder mehreren vorstehenden Ansprüchen, wobei der Prozessor ferner dazu konfiguriert ist, als Reaktion auf das Empfangen einer Suchanforderung, die einer bestimmten biologischen Probe zugeordnet ist, den Fortschritt der Analyse der bestimmten Probe zu bestimmen und eine Fortschrittsangabe über die grafische Benutzeroberfläche (214) zu erzeugen.

5. System nach einem oder mehreren vorstehenden Ansprüchen, wobei der Prozessor ferner dazu konfiguriert ist: das Vorhandensein einer ersten externen Qualitätskontrolle zu bestimmen, die eine Freigabe einer biologischen Komponente an eine Analysevorrichtung (432) für biologische Proben verhindert; und
als Reaktion auf die Bestimmung, dass die erste externe Qualitätskontrolle vorhanden ist, die Freigabe biologischer Komponenten an die Analysevorrichtung (432) für biologische Proben zu verhindern, um die Analyse der biologischen Komponenten durchzuführen, bis die erste externe Qualitätskontrolle freigegeben ist.

6. System nach Anspruch 5, wobei der Prozessor ferner konfiguriert ist zum:
Bestimmen des Vorhandenseins einer zweiten externen Qualitätskontrolle, die eine Freigabe der biologischen Komponenten nach Analyse durch die Analysevorrichtung (432) für biologische Proben verhindert; und
als Reaktion auf die Bestimmung, dass die zweite externe Qualitätskontrolle vorhanden ist, die Freigabe eines Analyseergebnisses für die biologischen Komponenten zu verhindern, bis die zweite externe Qualitätskontrolle freigegeben ist.

7. System nach einem oder mehreren vorstehenden Ansprüchen, wobei der Prozessor ferner dazu konfiguriert ist:
als Reaktion auf eine Bestimmung, dass ein potenzieller Pool die erste Komponente der ersten biologischen Probe, die dazu konfiguriert ist, mit mehreren Assays durch eine oder mehrere Analysevorrichtungen (432) für biologische Proben analysiert zu werden, und die zweite Komponente der zweiten biologischen Probe beinhaltet, die dazu konfiguriert ist, mit dem einzelnen Assay durch eine oder mehrere Analysevorrichtungen (432) für biologische Proben analysiert zu werden, zu verhindern, dass die erste oder zweite Komponente zu dem Pool hinzugefügt wird.

8. System nach einem oder mehreren vorstehenden Ansprüchen, wobei der Prozessor ferner dazu konfiguriert ist:
als Reaktion auf eine Bestimmung, dass ein Pool reaktiv ist, den Pool derselben oder einer anderen Analysevorrichtung für biologische Proben bereitzustellen, nachdem eine erste Analysevorrichtung (432) für biologische Proben die Analyse des Pools abgeschlossen hat, und/oder
als Reaktion auf eine Bestimmung, dass ein Pool reaktiv ist, den Pool zu dekonvolvieren, indem die Bestandteile dieses Pools derselben oder einer anderen Analysevorrichtung (432) für biologische Proben bereitgestellt werden, nachdem die erste Analysevorrichtung (432) für biologische Proben die Analyse des Pools abgeschlossen hat, und/oder
als Reaktion auf eine Bestimmung, dass ein Bestandteil in dem Pool reaktiv ist, den Bestandteil derselben oder einer anderen Analysevorrichtung (432) für biologische Proben bereitzustellen, nachdem die Analyse des Bestandteils des Pools abgeschlossen ist.

9. System nach einem oder mehreren vorstehenden Ansprüchen, wobei die biologischen Proben unter der Vielzahl von Zentrifugen (426), der Vielzahl von Pooling-Vorrichtungen (430) und der Vielzahl von Analysevorrichtungen (432) für biologische Proben automatisch über ein Schienensystem transportiert werden.

10. System nach einem oder mehreren vorstehenden Ansprüchen, wobei der Prozessor ferner dazu konfiguriert ist: einen Arbeitsablauf einer oder mehrerer der Vielzahl biologischer Proben basierend auf einem unerwarteten Resultat eines oder mehrerer des überwachten Vorgangs der Vielzahl von Zentrifugen (426), der Vielzahl von Pooling-Vorrichtungen (430) oder der Vielzahl von Analysevorrichtungen (432) für biologische Proben oder den verfolgten Analyseprozess der Vielzahl biologischer Proben automatisch einzustellen.

11. System nach Anspruch 10, wobei der Prozessor ferner dazu konfiguriert ist, den Arbeitsablauf basierend auf einer Lieferplanung für biologische Probe automatisch einzustellen.

12. System nach Anspruch 10, wobei der Prozessor ferner dazu konfiguriert ist, den Arbeitsablauf basierend auf einer oder mehreren einer Verfügbarkeit der Vielzahl von Zentrifugen (426), der Vielzahl von Pooling-Vorrichtungen (430), der Vielzahl von Analysevorrichtungen (432) für biologische Proben, einer Vielzahl von Reagenzien oder einer Vielzahl von Technikern automatisch einzustellen.

13. System nach einem oder mehreren vorstehenden Ansprüchen, wobei der Prozessor ferner dazu konfiguriert ist:
eine Fehlermeldung von einer oder mehreren der Vielzahl von Zentrifugen (426), der Vielzahl von Pooling-Vorrichtungen (430) oder der Vielzahl von Analysevorrichtungen (432) für biologische Proben zu empfangen;
eine Fehlermeldungsangabe über die grafische Benutzeroberfläche (214) zu erzeugen, wobei die Fehlermeldung einen oder mehrere eines Hardwarefehlers oder eines Softwarefehlers beinhaltet, und wobei der Arbeitsablauf basierend auf der Fehlermeldung automatisch weiter eingestellt wird.

14. Verfahren zum Analysieren biologischer Proben in einem System nach einem der Ansprüche 1 bis 13, wobei das Verfahren die folgenden Schritte umfasst:
Trennen durch die Vielzahl von Zentrifugen (426) mindestens einiger biologischer Proben aus der Vielzahl biologischer Proben in eine Vielzahl von Komponenten,
Poolen durch die Vielzahl von Pooling-Vorrichtungen (430) einer Vielzahl von Teilmengen der Vielzahl von Komponenten in eine Vielzahl gepoolter biologischer Komponenten, und
Analysieren durch die Vielzahl von Analysevorrichtungen (432) für biologische Proben der Vielzahl biologischer Proben und/oder der Vielzahl gepoolter biologischer Komponenten, um das Vorhandensein einer oder mehrerer Anomalien zu bestimmen;
wobei der Prozessor ferner angepasst ist zum:
Kommunizieren und Steuern einer Vielzahl von Zentrifugen (426), einer Vielzahl von Pooling-Vorrichtungen (430) und einer Vielzahl von Analysevorrichtungen (432) für biologische Proben;
Überwachen des Betriebs der Vielzahl von Zentrifugen (426), Vielzahl von Pooling-Vorrichtungen (430) und Vielzahl von Analysevorrichtungen (432) für biologische Proben und Erzeugen mindestens einer Warnung oder einer Störungsangabe über eine grafische Benutzeroberfläche (214);
Verfolgen des Fortschritts der Analyse einer Vielzahl biologischer Proben und Erzeugen einer Vielzahl von Fortschrittsangaben über die grafische Benutzeroberfläche (214); und
Speichern mindestens einiger der verfolgten Fortschritte in dem Speicher,
wobei der Prozessor ferner konfiguriert ist zum:
Bestimmen, ob einer oder mehrere der Vorgänge der Vielzahl von Pooling-Vorrichtungen (430) und der Vielzahl von Analysevorrichtungen (432) für biologische Proben an biologischen Proben ausgeführt werden, die unbeabsichtigte Duplikate sind;
als Reaktion auf das Bestimmen, dass der eine oder die mehreren der Vorgänge der Vielzahl von Pooling-Vorrichtungen (430) und der Vielzahl von Analysevorrichtungen (432) für biologische Proben an biologischen Proben durchgeführt werden, die unbeabsichtigte Duplikate sind, den Arbeitsablauf des einen oder der mehreren Vorgänge der Vielzahl von Zentrifugen (426), Vielzahl von Pooling-Vorrichtungen (430) und Vielzahl von Analysevorrichtungen (432) für biologische Proben, die an biologischen Proben durchgeführt werden, automatisch zu ändern,
wobei die Duplikate eine oder mehrere biologische Proben sind, die bereits Teil eines anderen Pools sind, der für dieselbe Analysevorrichtung (432) für biologische Proben geplant ist, oder wobei die Duplikate eine oder mehrere biologische Proben beinhalten, die nicht dieselbe Pooling-Anforderung wie eine andere biologische Probe in demselben Pool aufweisen.

15. Verfahren nach einem oder mehreren vorstehenden Ansprüchen, wobei das Verfahren ferner als Reaktion auf das Empfangen einer Suchanforderung, die einer bestimmten biologischen Probe zugeordnet ist, das Bestimmen des Analysefortschritts der bestimmten biologischen Probe und das Erzeugen einer Fortschrittsangabe über die grafische Benutzeroberfläche (214) umfasst.

16. System nach einem oder mehreren vorstehenden Ansprüchen, wobei das Verfahren ferner Folgendes umfasst:
Anzeigen einer Liste von Elementen, wobei die Liste von Elementen eines oder mehrere der folgenden umfasst:
eine oder mehrere Aktionen für einen Bediener, wobei die eine oder die mehreren Aktionen Folgendes umfassen: Nachfüllen von Spitzen an einem oder mehreren Instrumenten, Nachfüllen von Reagenzien, Entleeren von Abfall, Laden von Probencharge(n), Entladen abgeschlossener Proben oder Zubereiten von Reagenzien;
eine Dauer für die Aktion; oder
eine Instrumentenkennung;
wobei die Liste von Elementen durch eine oder mehrere Messungen von Dringlichkeit der zugeordneten Aktionen sortiert wird.

17. Verfahren nach einem oder mehreren vorstehenden Ansprüchen, wobei die Liste der Elemente eine eindeutige Kennung eines oder mehrerer Instrumente, die Aktionen desselben Typs und derselben Dringlichkeit zugeordnet sind, umfasst.

## Revendications

1. Système (200) pour l'analyse d'échantillons biologiques, le système comprenant :
une pluralité de centrifugeuses (426) configurées pour séparer au moins certains échantillons biologiques d'une pluralité d'échantillons biologiques en une pluralité de composants ;
une pluralité de dispositifs de regroupement (430) adaptés pour regrouper une pluralité de sous-ensembles de la pluralité de composants en une pluralité de composants biologiques regroupés ;
une pluralité de dispositifs d'analyse d'échantillons biologiques (432) configurés pour analyser la pluralité d'échantillons biologiques et/ou la pluralité de composants biologiques regroupés afin de déterminer la présence d'une ou plusieurs anomalies ;
un processeur adapté pour communiquer avec la pluralité de centrifugeuses (426), la pluralité de dispositifs de regroupement (430) et la pluralité de dispositifs d'analyse d'échantillons biologiques (432) et pour les commander, le processeur étant en outre adapté pour :
surveiller les opérations de la pluralité de centrifugeuses (426), de la pluralité de dispositifs de regroupement (430), et de la pluralité de dispositifs d'analyse d'échantillons biologiques (432) et générer au moins l'une des indications d'avertissement ou de défaillance par l'intermédiaire d'une interface utilisateur graphique (214) ;
suivre la progression de l'analyse de la pluralité d'échantillons biologiques et générer une pluralité d'indications de progression par l'intermédiaire de l'interface utilisateur graphique (214) ; et
stocker au moins une partie des progrès suivis dans la mémoire,
dans lequel le processeur est en outre configuré pour :
déterminer si une ou plusieurs des opérations de la pluralité de dispositifs de regroupement (430) et de la pluralité de dispositifs d'analyse d'échantillons biologiques (432) sont effectuées sur des échantillons biologiques qui sont des doublons involontaires ;
en réponse à la détermination que l'une ou plusieurs des opérations de la pluralité de dispositifs de regroupement (430) et de la pluralité de dispositifs d'analyse d'échantillons biologiques (432) sont effectuées sur des échantillons biologiques qui sont des doublons involontaires, modifier automatiquement le flux de travail pour l'une ou plusieurs des opérations de la pluralité de centrifugeuses (426), de la pluralité de dispositifs de regroupement (430) et de la pluralité de dispositifs d'analyse d'échantillons biologiques (432) qui sont effectuées sur des échantillons biologiques qui sont des doublons involontaires,
dans lequel les doublons sont un ou plusieurs échantillons biologiques qui font déjà partie d'un autre regroupement programmé pour le même dispositif d'analyse d'échantillons biologiques (432), ou dans lequel les doublons comprennent un ou plusieurs échantillons biologiques qui n'ont pas la même exigence de regroupement qu'un autre échantillon biologique dans le même regroupement.

2. Système de la revendication 1, dans lequel la pluralité d'échantillons biologiques comprend des échantillons de sang et/ou de plasma.

3. Système de l'une ou de plusieurs des revendications précédentes, dans lequel la pluralité de dispositifs d'analyse d'échantillons biologiques est configurée pour effectuer au moins l'un des tests d'amplification de l'acide nucléique (TAN), l'immunohématologie ou la sérologie.

4. Système de l'une ou de plusieurs des revendications précédentes, dans lequel le processeur est en outre configuré pour, en réponse à la réception d'une demande de recherche associée à un échantillon biologique particulier, déterminer la progression de l'analyse de l'échantillon particulier et générer une indication de progression par l'intermédiaire de l'interface utilisateur graphique (214).

5. Système de l'une ou de plusieurs des revendications précédentes, dans lequel le processeur est en outre configuré pour :
déterminer la présence d'un premier contrôle de qualité externe empêchant la libération d'un composant biologique dans un dispositif d'analyse d'échantillons biologique (432) ; et
en réponse à la détermination de la présence du premier contrôle de qualité externe, empêcher la libération de composants biologiques vers le dispositif d'analyse d'échantillons biologiques (432) pour effectuer l'analyse des composants biologiques jusqu'à ce que le premier contrôle de qualité externe soit libéré.

6. Système de la revendication 5, dans lequel le processeur est en outre configuré pour :
déterminer la présence d'un deuxième contrôle de qualité externe empêchant la libération des composants biologiques après analyse par le dispositif d'analyse d'échantillons biologiques (432) ; et
en réponse à la détermination de la présence du deuxième contrôle de qualité externe, empêcher la libération d'un résultat d'analyse pour les composants biologiques jusqu'à ce que le deuxième contrôle de qualité externe soit libéré.

7. Système de l'une ou de plusieurs des revendications précédentes, dans lequel le processeur est en outre configuré pour :
en réponse à la détermination qu'un regroupement potentiel comprend le premier composant du premier échantillon biologique configuré pour être analysé avec plusieurs essais par un ou plusieurs dispositifs d'analyse d'échantillons biologiques (432) et le deuxième composant du deuxième échantillon biologique configuré pour être analysé avec l'essai unique par un ou plusieurs dispositifs d'analyse d'échantillons biologiques (432), empêcher le premier ou le deuxième composant d'être ajouté au regroupement.

8. Système de l'une ou de plusieurs des revendications précédentes, dans lequel le processeur est en outre configuré pour :
en réponse à la détermination qu'un regroupement est réactif, fournir le regroupement au même ou à un autre dispositif d'analyse d'échantillons biologiques après qu'un premier dispositif d'analyse d'échantillons biologiques (432) ait terminé l'analyse du regroupement, et/ou
en réponse à la détermination qu'un regroupement est réactif, déconvoluer le regroupement en fournissant les constituants de ce regroupement au même ou à un autre dispositif d'analyse d'échantillons biologiques (432) après que le premier dispositif d'analyse d'échantillons biologiques (432) ait terminé l'analyse du regroupement, et/ou en réponse à la détermination qu'un constituant du regroupement est réactif, fournir le constituant au même ou à un autre dispositif d'analyse d'échantillons biologiques (432) après que l'analyse du constituant du regroupement est terminée.

9. Système de l'une ou de plusieurs des revendications précédentes, dans lequel les échantillons biologiques sont transportés entre la pluralité de centrifugeuses (426), la pluralité de dispositifs de regroupement (430) et la pluralité de dispositifs d'analyse d'échantillons biologiques (432) automatiquement par l'intermédiaire d'un système de suivi.

10. Système de l'une ou de plusieurs des revendications précédentes, dans lequel le processeur est en outre configuré pour :
ajuster automatiquement un flux de travail d'un ou de plusieurs de la pluralité d'échantillons biologiques sur la base d'un résultat inattendu d'un ou de plusieurs des éléments suivants : l'opération surveillé de la pluralité de centrifugeuses (426), la pluralité de dispositifs de regroupement (430), ou la pluralité de dispositifs d'analyse d'échantillons biologiques (432), ou la progression suivie de l'analyse de la pluralité d'échantillons biologiques.

11. Système de la revendication 10, dans lequel le processeur est en outre configuré pour ajuster automatiquement le flux de travail en fonction d'un calendrier de livraison d'échantillons biologiques.

12. Système selon la revendication 10, dans lequel le processeur est en outre configuré pour ajuster automatiquement le flux de travail en fonction d'un ou de plusieurs éléments parmi la disponibilité de la pluralité de centrifugeuses (426), la pluralité de dispositifs de regroupement (430), la pluralité de dispositifs d'analyse d'échantillons biologiques (432), une pluralité de réactifs, ou une pluralité de techniciens.

13. Système de l'une ou de plusieurs des revendications précédentes, dans lequel le processeur est en outre configuré pour :
recevoir un message d'erreur provenant d'un ou plusieurs de la pluralité de centrifugeuses (426), la pluralité de dispositifs de regroupement (430), ou la pluralité de dispositifs d'analyse d'échantillons biologiques (432) ; et
générer une indication de message d'erreur via l'interface utilisateur graphique (214), dans lequel le message d'erreur comprend une ou plusieurs erreurs matérielles ou une erreur logicielle et dans lequel le flux de travail est automatiquement ajusté davantage sur la base du message d'erreur.

14. Procédé d'analyse d'échantillons biologiques dans un système selon l'une quelconque des revendications 1 à 13, le procédé comprenant les étapes consistant à :
séparer, par la pluralité de centrifugeuses (426), au moins certains échantillons biologiques de la pluralité d'échantillons biologiques en une pluralité de composants, regrouper, par la pluralité de dispositifs de regroupement (430), une pluralité de sous-ensembles de la pluralité de composants en une pluralité de composants biologiques regroupés, et
analyser, par la pluralité de dispositifs d'analyse d'échantillons biologiques (432), la pluralité d'échantillons biologiques et/ou la pluralité de composants biologiques regroupés pour déterminer la présence d'une ou plusieurs anomalies.
dans lequel le processeur est adapté pour
communiquer avec et contrôler une pluralité de centrifugeuses (426), une pluralité de dispositifs de regroupement (430), et une pluralité de dispositifs d'analyse d'échantillons biologiques (432) ;
surveiller les opérations de la pluralité de centrifugeuses (426), de la pluralité de dispositifs de regroupement (430) et de la pluralité de dispositifs d'analyse d'échantillons biologiques (432) et générer au moins un avertissement ou une indication de défaillance par l'intermédiaire d'une interface utilisateur graphique (214) ;
suivre la progression de l'analyse d'une pluralité d'échantillons biologiques et générer une pluralité d'indications de progression par l'intermédiaire de l'interface utilisateur graphique (214) ; et
stocker au moins une partie des progrès suivis dans la mémoire,
dans lequel le processeur est en outre configuré pour :
déterminer si une ou plusieurs des opérations de la pluralité de dispositifs de regroupement (430) et de la pluralité de dispositifs d'analyse d'échantillons biologiques (432) sont effectuées sur des échantillons biologiques qui sont des doublons involontaires ;
en réponse à la détermination que l'une ou plusieurs des opérations de la pluralité de dispositifs de regroupement (430) et de la pluralité de dispositifs d'analyse d'échantillons biologiques (432) sont effectuées sur des échantillons biologiques qui sont des doublons involontaires, modifier automatiquement le flux de travail pour l'une ou plusieurs des opérations de la pluralité de centrifugeuses (426), de la pluralité de dispositifs de regroupement (430) et de la pluralité de dispositifs d'analyse d'échantillons biologiques (432) qui sont effectuées sur des échantillons biologiques qui sont des doublons involontaires,
dans lequel les doublons sont un ou plusieurs échantillons biologiques qui font déjà partie d'un autre regroupement programmé pour le même dispositif d'analyse d'échantillons biologiques (432), ou dans lequel les doublons comprennent un ou plusieurs échantillons biologiques qui n'ont pas la même exigence de regroupement qu'un autre échantillon biologique dans le même regroupement.

15. Procédé d'une ou de plusieurs revendications précédentes, dans lequel le procédé comprend en outre, en réponse à la réception d'une demande de recherche associée à un échantillon biologique particulier, la détermination de la progression de l'analyse de l'échantillon biologique particulier et la génération d'une indication de progression par l'intermédiaire de l'interface utilisateur graphique (214).

16. Procédé selon l'une ou plusieurs des revendications précédentes, dans lequel le procédé comprend en outre : l'affichage d'une liste d'éléments, la liste d'éléments comprenant un ou plusieurs :
une ou plusieurs actions pour un opérateur, ladite ou lesdites actions consistant à :
recharger des pointes d'un ou plusieurs instruments, recharger des réactifs, vider des déchets, charger un/des lot(s) d'échantillons, décharger des échantillons terminés, ou préparer des réactifs ;
une durée pour l'action ; ou
un identifiant d'instrument,
dans lequel la liste des éléments est triée en fonction d'une ou plusieurs mesures de l'urgence des actions associées.

17. Procédé selon l'une ou plusieurs des revendications précédentes, dans lequel la liste d'éléments comprend un identifiant unique pour un ou plusieurs instruments associés à des actions du même type et de la même urgence.
